Europäisches Patentamt

(19) European Patent Office

Office européen des brevets

(11) Publication number: **0 125 393**
**B1**

(12) **EUROPEAN PATENT SPECIFICATION**

(45) Date of publication of patent specification: **09.12.87**

(51) Int. Cl.⁴: **A 61 F 2/24**

(21) Application number: **84101480.6**

(22) Date of filing: **29.10.81**

(80) Publication number of the earlier application in accordance with Art. 76 EPC: **0 051 451**

(54) Prosthetic heart valve.

(30) Priority: **03.11.80 US 203840**

(43) Date of publication of application:
**21.11.84 Bulletin 84/47**

(45) Publication of the grant of the patent:
**09.12.87 Bulletin 87/50**

(84) Designated Contracting States:
**BE CH DE FR GB IT LI NL SE**

(56) References cited:
DE-B-2 355 959
FR-A-2 451 189
GB-A-1 264 472
US-A-3 197 788
US-A-3 714 671
US-A-3 739 402
US-A-3 983 581
US-A-4 079 468
US-A-4 084 268

(73) Proprietor: **SHILEY INCORPORATED**
**17600 Gillette**
**Irvine California (US)**

(72) Inventor: **Ionescu, Marian Ian**
**357 Street Lane**
**Leeds L517 6RU (GB)**
Inventor: **Lenker, Jay Alan**
**259 Fairview Street**
**Laguna Beach California (US)**
Inventor: **Rosenbluth, Robert Frank**
**24591 La Hermosa Avenue**
**Laguna Niguel California (US)**
Inventor: **Seiler, Louis, Jr.**
**17341 Forbes Lane**
**Huntington Beach California (US)**

(74) Representative: **Wood, David John et al**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The early development of prosthetic heart valves is well documented in papers given at symposia in 1960 and in 1968, published in Prosthetic Heart Valves, Lyman A. Brewer III, Ed., Charles C. Thomas Publishing Co., Springfiled, Illinois (1969), Second National Conference on Prosthetic Heart Valves; Prosthetic Valves for Cardiac Surgery, K. Alvin Merendino, Ed., Charles C. Thomas Publishing Co., Springfield, Illinois (1961).

Lefrak and Starr recently surveyed the development of cardiac valve prostheses, E. A. Lefrak and A. Starr, Cardiac Valve Prostheses, Appleton-Century-Krofts, New York, 1979 and the development of tissue heart valves has been comprehensively reviewed by Ionescu, Marian I, Tissue Heart Valves, Butterworths, Boston, 1979.

Great efforts have been expended in the development of tissue heart valve prostheses and in the development of supportive structures, or stents, for tissue valves. Representative of effects to develop stents for tissue valves are the disclosures in the following United States patents: Patent 3,570,014, W. D. Hancock, March 16, 1971; Patent 3,714,671, William Sterling Edwards et al, February 6, 1973; Patent 3,755,823, W. D. Hancock, September 4, 1973; Patent 3,983,581, William W. Angell, October 5, 1976; Patent 4,035,849, William W. Angell et al, July 19, 1977; Patent 4,079,468, Domingo Santo Liotta, March 21, 1978; Patent 4,084,268, Marian I. Ionescu et al., April 18, 1978; Patent 4,106,129, Alain F. Carpentier et al, August 15, 1978; Patent 4,172,295, Richard J. Batten, October 30, 1979 and Patent 4,192,020, Richard B. Davis et al, March 11, 1980. Other structures are also reported in the aforementioned treatises on heart valve developments.

A number of specific tissue valves are described in the following publications:

W. Sterling Edwards et al, Mitral and Aortic Valve Replacement with Fascia Lata on a Frame, *Journal of Thoracic & Cardiovascular Surgery, Volume 58, No. 6,* December 1969, Pages 854—858; Ionescu, M. I. et al, Heart Valve Replacement with Ionescu-Shiley Pericardial Xenograft, *Cardiology Digest*, June 1977, Page 45; Ionescu, M. I. et al, Heart Valve Replacement with the Ionescu-Shiley Pericardial Xenograft, *The Journal of Thoracic and Cardiovascular Surgery, Volume 73*, Pages 31—42, 1977; Tandon, A. P. et al, Long-Term Haemodynamic Evaluation of Aortic Pericardial Xenograft, *British Heart Journal, Volume 40*, Pages 602—607, 1978; Ionescu, M. I. et al. Long-Term Clinical and Haemodynamic Evaluation of the Ionescu-Shiley Pericardial Xenograft Heart Valve, *Thoraxchirugie, Volume 26,* Pages 250—258, 1978; Ionescu, M. I. et al. Long-Term Sequential Hemodynamic Evaluation of Right Ventricular Outflow Tract Reconstruction Using a Valve Mechanism, *The Annals of Thoracic Surgery, Volume 27, No. 5,* May 1979; Ross, D. N., Flexible Bioprosthetic Pericardial Heart Valve, *Thoracic & Cardiovascular Surgery, Volume 28,* Pages 150—152, 1980.

Particular techniques for preparing, handling and storing tissue valves are disclosed in U.S. Patents Nos. 3,966,401, Hancock et al. June 29, 1976, and 4,182,446, Penny, January, 1980.

Some of the earliest heart valve prostheses were flexible two-or three-cusp valves in which the cusps were constructed of various types of fabric. Some of these flexible leaflet valves had good flow characteristics but most failed early. The leaflets tore, separated from the annulus, or become rigid due to fibrous tissue ingrowth. From about 1960 into the 1970's the trend was to mechanical valves. These ranged from the mechanically quite simple Starr-Edwards valve to the relatively sophisticated Bjork-Shiley valve and included a number of disc poppet valves. These mechanical valves generally dominated the market and are still very satisfactory for many applications. Tissue valves are however the preferred treatment where anticoagulation therapy is not tolerated by the patient.

In 1962, Donald Ross and Sir Brian Barrat-Boyes, working independently, were performing implantations of homograft tissue valves, some of which were free graft implants and some of which were mounted on supporting stents. Fully clothed covered rigid stents were used in some of these homograft valves.

In 1965, Drs. Binet and Carpentier, and their associates, implanted a specially prepared porcine aortic valve xenograft. These porcine valves were sterilized and treated, e.g. with formaldehyde, and were commonly attached to a metal stent. Experience showed that these valves were of short life, largely because formaldehyde was used as the cross-linking agent. Formaldehyde was found to create reversible cross links in the tissue, thereby allowing early breakdown of the tissue. Dr. Carpentier, in about 1968, established the concept of the bioprosthesis by substantially eliminating antigenicity of the tissue, principally by changing the preservative from formaldehyde to glutaraldehyde. Glutaraldehyde has been shown to create cross links of a more permanent nature than those created by formaldehyde.

A number of porcine bioprostheses and specially designed stents for supporting these prostheses resulted from the efforts of Warren Hancock et al. Generally, pig aortic valves are procured under clean conditions, placed in a cold, balanced electrolyte solution, excess tissue is trimmed and the xenografts are immersed in 0.2% glutaraldehyde. The leaflets are held in their normal valving position under pressure during the tanning process and each valve is sutured to a cloth covered stent by sutures. A number of designs and stent constructions for the Hancock type valve are exemplified in the aforementioned United States Patents Nos. 3,570,014 and 3,755,823. Stents for porcine valves were developed by a number of other workers also, see, e.g., U.S. Patents Nos. 3,983,581; 4,035,849; 4,079,468 and 4,106,129.

Stents for supporting cusp valves of other tissue members, e.g. fascia lata and pericardium, have been developed by a number of workers, see, e.g., U.S. Patent 3,714,671, and Edwards et al, Mitral and Aortic Valve Replacement with Fascia Lata on a Frame, supra. Much of the pioneering work in this area of valve development was done by Dr. Martin I. Ionescu and his associates, see, e.g., Bartek et al, Frame-Mounted Tissue Heart Valves: Technique of Construction, *Thorax, Volume 29,* Pages 51—55, 1974; Ionescu et al, Heart Valve Replacement with Ionescu-Shiley Pericardial Xenograft, *Cardiology Digest,* June 1977; Ionescu et al, Heart Valve Replacement with Ionescu-Shiley Pericardial Xenograft, *The Journal of Thoracic and Cardiovascular Surgery, Volume 73,* Pages 31—42, 1977; Tandon et al, Long-Term Haemodynamic Evaluation of Aortic Pericardial Xenograft, *British Heart Journal,* Volume 40, Pages 602—607, 1978; Ionescu et al, Long-Term Clinical and Haemodynamic Evaluation of the Ionescu-Shiley Pericardial Xenograft Heart Valve, *Thoraxchirugie, Volume 26,* Pages 250—258, 1978; Ionescu, et al, Long-Term Sequential Hemodynamic Evaluation of Right Ventricular Outflow Tract Reconstruction Using a Valve Mechanism, *The Annals of Thoracic Surgery, 27,* 425—434, 1979; and Ionescu, Editor, Tissue Heart Valves, Butterworths, 1979.

A number of improvements in the basic Ionescu tissue heart valve have been made. For example, a tissue heart valve has been developed which has a cloth-covered stent of special construction, in which the outflow annulus diameter of the valve is defined and limited by the positioning of a coaptation stitch on the inside of the supporting legs of the stent, as has been the practice since the early development of the Ionescu type tissue heart valve. Another improvement in the method for aligning the tissue of the cusps of the Ionescu type heart valve is described in U.S. Patent No. 4,172,295, which also discloses the coaptation stitch inside the stent legs. It has been the practice, in order to achieve a maximum flow orifice in valves of implantation diameters less than or equal to 23 mm, to splay the stent legs outwardly in an effort to achieve a full-flow orifice inside the coaptation stitches.

While these various modifications and improvements in the basic Ionescu, valve over the years have solved some of the problems, there remains a number of problems which have not been solved. Among these problems are the limitations on the size of the flow annulus which can be obtained, with consequent increased pressure gradient across the valve. Perhaps the most important disadvantage of the three-cusp tissue valves of the prior art is the fact that in each cusp there are two points of stress due to three-dimensional flexure at about 4 o'clock and at about 8 o'clock in the lower arc of the cusp, i.e., in the lower right hand portion and the lower left hand portion of the closed cusp as viewed laterally, head-on, from the outside. A similar phenomena is created by creases which tend to concentrate stress in the areas of the crease.

The tissue valves of the prior art generally have relied upon a face-to-face meeting of the cusps in the closed position and, consequently, have required relatively high stent legs and, in general, the tissue valves of the prior art have trimmed the tissue around the outflow end so as to form a generally round regular cylindrical configuration. This approach has resulted in the concentration of stresses in fold areas or in the lower right and left hand portions of the cusps.

An additional potential problem is that stress is concentrated in the tissue in some areas where sharp bending of the tissue around the stent occurs. Some measures can be taken to relieve this stress. The stress tends to be highest at points of maximum curvature such as around the tips of the stent legs because of the pinching of the tissue leaflets together inside and above the tip of the stent leg.

The present invention comprises an improved prosthetic heart valve. The prosthetic heart valve of this invention comprises:

(a) a stent (102) comprising:

(1) an annular ring (104) having inflow and outflow edges and defining a flow aperture; and
(2) a plurality of legs (106, 108, 110) coupled to said ring (104) and extending from said outflow edge in the direction of flow, with neighboring pairs of said legs (106, 108, 110) being separated by scallops (112) formed in said outflow edge, which scallops (112) are concave towards the direction of flow;

(b) a plurality of tissue leaflets (30, 50, 70) attached to said stent (102) and to each other to form a valving element (20); and
(c) a plurality of coaptation means (130, 132, 132) in the vicinity of the tips (107, 109, 111) of said stent legs (106, 108, 110) for causing joining of the outflow edges (40, 60, 80) of said tissue leaflets (30, 50, 70), characterized in that said stent (102) is dimensioned such that the quotient of the radius of curvature of mobile tissue (21) divided by the thickness of said tissue (21) is greater than or equal to five at all points of contact between said mobile tissue (21) and said stent (102).

Preferably, the heart valve is further characterised in that said valve includes inwardly projecting tab means (120, 123, 125) at the outflow end of each stent leg (106, 108, 110) for adding structural strength to said legs (106, 108, 110), with the distance that said tab means (120, 123, 125) project inwardly toward the center of said flow aperture being restricted to a distance (P) which substantially eliminates abrasion between the tissue leaflets (30, 50, 70) and the bottom surfaces (142) of said tab means (120, 123, 125).

Additionally, the valve is preferably further characterised in that said valve includes means on said tab means (120, 123, 125) coupled to said coaptation means (130, 132, 134) for fixing the position of said coaptation means (130, 132, 134), with said means for fixing the positions of said coaptation means comprising a plurality of holes (121) through said tab means (120, 123, 125).

3

**0 125 393**

This application is a divisional of application No. 81305122.4 (0051451) which describes and claims a prosthetic heart valve wherein the leaflets have a particular shape.

For convenience, in describing the valve, the outflow end of the valve is depicted at the top of the drawings and the valve is described in this configuration; thus, the upward or top portion of the valve would correspond to the outflow portion of the valve and the bottom would correspond to the inflow end of the valve.

In the prosthetic valve of this invention, the scallop depth U of the stent is preferably between 50% and 65% of the stent inner diameter D. Optimally, the stent scallop depth U is from about 55% to about 62% of the diameter D. This U/D ratio provides a low profile tissue valve prosthesis which has optimum flexure and coaptation of the leaflets using tissue fixed in the unstressed state, and resultant maximum valve life.

A significant feature of the present invention resides in the configuration of the stent legs wherein the ratio defined as the value of the radius of curvature of tissue around the inside top of said leg measured at the point of greatest curvature divided by the thickness of the tissue is five or more. The width of the post top is preferably approximately from 0.760 to 1.05 millimeters and the thickness of the cloth covered post top is preferably approximately 2 millimeters. The tissue thickness in the preferred embodiment is approximately 0.3 millimeters. The tissue does not pinch together inside such narrow posts during closure at physiological pressure and an inside radius of curvature equal to or greater than 1.5 millimeters is obtained. The life of a valve using such a stent will be extended beyond that of a valve with a smaller radius of curvature.

The stent of the improved heart valve is designed so that the ratio of the radius of curvature of the tissue around the stent at a point between the up-right stent legs divided by the thickness of the tissue is greater than or equal to five.

Additional strength to the legs is provided by inwardly projecting tabs which are, preferably, integrally formed at the tips of the legs, conforming to the width of the stent legs at their tips but narrower than the stent legs at the bottom of the tabs, said tabs extending both vertically down the leg for a predetermined distance and inwardly toward the center axis of the stent. Apertures are formed in the tabs through which the coaptation stitches pass to register the coaptation stitch directly above the stent leg.

The coaptation stitches can be in either of two embodiments. The first embodiment consists of two separate stitches, one passing through one aperture in the tab and defining a plane generally parallel to the long axis of the stent legs and tied off above the tips of the stent legs, and the other stitch passing through another aperture in the tabs and defining a plane which is generally perpendicular to the plane defined by the first stitch, said second stitch being tied off at the outside edge of the stent leg. The second embodiment of the coaptation stitch is a single stitch passing through a plurality of holes in the tabs which defines a plane which is parallel to the long axis of the stent leg. The second embodiment is the preferred embodiment and generally defines a figure eight pattern through a pair of holes in each tab.

The inward projection of the tabs toward the center of the flow aperture of the valve is also limited to prevent touching between the tissue leaflets and the bottom of the tabs. Generally this distance of inward projection is limited to forty thousandths of an inch (1.016 mm) in the naked stent, i.e., no more than forty thousandths inward projection of the bare plastic tab. The width of the tabs is also limited to aid in preventing the bottoms of the tabs from touching the tissue leaflets. In the preferred embodiment, the widths of the tabs remains constant throughout their length while the widths of the stent legs increases at points farther from the tips of the stent legs. In another embodiment, the widths of the tabs decrease at points further from the tips of the stent legs irrespective of the widths of the stent legs.

Figure 1 is a perspective view of a completed valve of this invention.

Figure 2 is an exploded view of the cloth covered stent and the valving element formed of three leaflets prior to attachment to the stent.

Figure 3 is a perspective view of the preferred stent configuration.

Figure 4 is a sectional view of the curvature of tissue around the covered stent leg taken along line 4—4 in Figure 2.

Figure 5 is a top view of the stent shown in perspective in Figure 3.

Figure 6 is a sectional view taken along section line 6—6 in Figure 5.

Figure 7 is a partial perspective view of the coaptation of the tissue leaflets with the stent leg and coaptation stitch.

Figure 8 is another partial perspective view showing the view of Figure 7 in a later stage of manufacture.

Figure 9 is a top plan view, viewed from the outflow end, of the completed valve in the fully open position.

Figure 10 is a sectional view of the curvature of tissue around the stent taken along section lone 10—10 in Figure 2.

Figure 11 is a detailed view of an embodiment of the coaptation stitching.

Figure 12 is a detailed view of the preferred embodiment of the coaptation stitching.

Figure 13 is a detailed view of a typical tab and stent leg tip showing the relative widths of each.

Figure 14 is a detailed view of another embodiment of a typical tab and stent leg tip showing the relative widths of each.

Figure 15 is a top view and a sectional view, taken along the section line in the top view, of a prior art

4

stent utilized in one of the improvements of the basic Ionescu valve, with the measurements thereof shown for purposes of comparison with the present invention.

Figure 16 is a flat plan view of the outline of one of the leaflets preferably used in the present valving element, before the sewing of three such leaflets together to form a cylindrical valving member.

Figure 17 is a top plan view, viewed from the outflow end, of the completed valve in the fully closed position.

Figure 18 is a partial cross-section taken along line 18—18 in Figure 8 showing the closed cusps of the valve leaflets along the closure line.

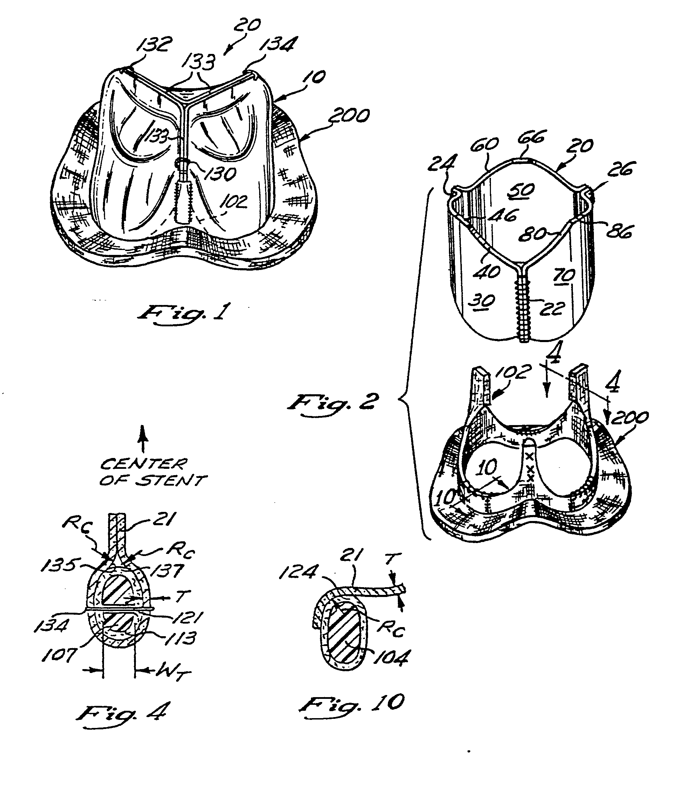

Figure 1 shows a low profile pericardial xenograft heart valve 10 which comprises a valving element 20, a stent assembly 102 and a suture or sewing ring assembly 200. The coaptation stitches 130, 132 and 134 cause coaptation or joining of the edges of the tissue leaflets of valving element 20 in the vicinity of the tips of the stent legs thereby forming radial coaptation lines 133 in Figure 1.

Figure 2 depicts an exploded view of the tissue valving element 20 prior to attachment of the stent 102, and generally shows the three stitch seams 22, 24 and 26 which join the three tissue leaflets 30, 50 and 70 into a right cylindrical valving element. This valving element is then sewn to the cloth-covered stent 102.

Figure 16 depicts a preferred tissue leaflet configuration. One of the leaflets, leaflet 30, of the valving element 20, is depicted as exemplary of all of the leaflets 30, 50 and 70, all of which are substantially identical. The leaflet 30 is a generally flat layer or sheet of pericardium tissue, treated as will be described and discussed in more detail hereinafter, and includes a curved bottom 32 and curved sides 34 and 36 with a top, or outflow edge generally indicated at 40. The top 40, however, comprises three distinct portions. Edges 42 and 44 converge upwardly like symmetrical sides of a triangle to a central plateau 46 at the top and form obtuse corners with the sides 34 and 36, respectively, on the bottom. Each of the leaflets 50 and 70 are to be understood as including corresponding elements, including the top or outflow edges 60 and 80, as shown in Figure 2, for example, and plateaus 66 and 86 corresponding to plateau 46. The three leaflets 30, 50 and 70 are in all essential respects identical, although there will be some minor variation in the exact shape and size of these leaflets because they are made from naturally occurring tissue and considerable manual dexterity and skill is required in production. Minor variations, so long as the function is not impaired, are readily tolerated in the present valve construction.

The precise dimensions of the leaflets 30, 50 and 70 are not critical because minor deviations and dimensions can be compensated for in the final joinder of the leaflets into the valving element and in fitting the valving element over the stent. One portion of the configuration of the leaflets is of considerable importance to the optimum functioning of the valve. This important portion of the configuration of the truncated triangular top edge converting to the plateau 46 in Figure 16 and to plateaus 66 and 86 in the leaflets 50 and 70. The base of the triangle is, of course, an imaginary line joining the lower corners of the top edge. Plateau 46 is centrally located between the juncture of the outflow edge portion 42 with side 34 and outflow edge portion 44 with side 36. The best definition of the shape of this obtuse truncated triangle defined by the plateau 46 and the juncture of the top edges 42 and 44 with the sides of the leaflet, is that this truncated triangle is so configured and dimensioned that the three leaflets are sewn together at their respective edges to form a cylinder, and fitted over a stent, with a coaptation stitch positioned directly over the end of the stent when the valve is closed, the plateau 46 of the leaflet 30 touches, or substantially touches, the corresponding plateaus 66 and 86 of the leaflets 50 and 70, with no more than about 1 mm of face-to-face contact, in the center of the flow path of the valve with substantial face-to-face contact, i.e. from about 2 mm to about 6 or 7 mm, between the interior surfaces of the edges intermediate the center and the outer diameter of the valve. The plateaus 46, 66 and 86 are in touching or substantially in touching relationship when the valve is closed, and there is substantial surface-to-surface contact along the central portion of the radial coaptation lines of cusp contact. This relationship is shown in Figure 18 which depicts the valve of Figure 1 cut perpendicular the radius defined by the cusp coaptation line 133, the area of contact being shown at 90. The maximum face-to-face contact is about halfway between the center and the legs and would be at least 2 or 3 mm but not more than 9 or 10 mm, optimally from 3 to 7 mm depending on valve size.

It is neither necessary nor possible to give exact shape and dimensional definitions to the preferred leaflets exemplified by leaflet 30, but the configuration may be described, realizing that the truly important relationship is the interrelationship of the three obtuse truncated triangular portions. The maximum width of the leaflet lies about midheight thereof. The height of the leaflet is, of course, of no criticality whatever, and so this is merely a general relationship. Thus, the sum of Sa, Sb and Sc (see Figure 16) is approximately equal to one-half of the total vertical height of the leaflet, Sa representing the mean altitude of the obtuse truncated triangle formed by plateau 46, converging edge portions 42 and 44 and the base defined by the junctures of top edge 40 with side 34 and side 36, respectively, Sb plus Sa being equal to about 35% plus or minus 3 to 5% of the total vertical height, and the sum of Sa, Sb and Sc being about 50% plus or minus around 10% of the total vertical height. The width of the leaflet, Wa, measured Sa down from the plateau 46 is about 85% plus or minus 10% of the maximum width, Wc, of the leaflet, Sa being around 12 to 17% of the total vertical height. The width Wb measured at Sa plus Sb from the plateau 46 is about 95% plus or minus about 5% of the maximum width. The exact width and height ratios depend generally upon the overall size of the valve and will usually fall within the ranges indicated, although the first definition by function is the best and most meaningful description presently comprehended. In a specific embodiment, the valving member for the size 23 valve is a section of pericardium 0.012 inch (0.305 mm) thick, with a maximum height of 21 millimeters and a maximum width Wc of 26.5 millimeters at about 52% of total height. The width Wa of the obtuse triangle is 22.5 millimeters measured at Sa down about 14% of the total height from the top, the intermediate width Wb being 25.5 millimeters at Sb, 35% from the top. Again, this is merely one example of one size of a valve and the dimensions are not the important factor; it is the interrelationship of the top edges of the leaflet that is important in the preferred leaflet configuration.

Glutaraldehyde has been used effectively to stabilize connective tissue for clinical heart valve substitutes for several years. The tissue leaflets of the present invention are cut from pericardium tissue, although other tissues may be used. The use of formaldehyde and glutaraldehyde tanning in preservation of tissue is described by E. Aubrey Woodruff, *The Chemistry and Biology of Aldehyde Treated Tissue Heart Valve Xenografts*, in Ionescu, Tissue Heart Valves, Butterworths, 1979. Woodruff and other contributors to Tissue Heart Valves discuss in detail the glutaraldehyde tanning and preservation of connective tissue. This disclosure is incorporated herein by reference. In the preferred embodiment of the present invention, pericardium treated with .5% glutaraldehyde at pH 7.4 without fixing the tissue in a prestressed condition is preferred; however, it is to be understood that the invention disclosed and claimed herein relates primarily to the configuration of the supporting stent, and any suitably preserved tissue may be utilized in the present invention.

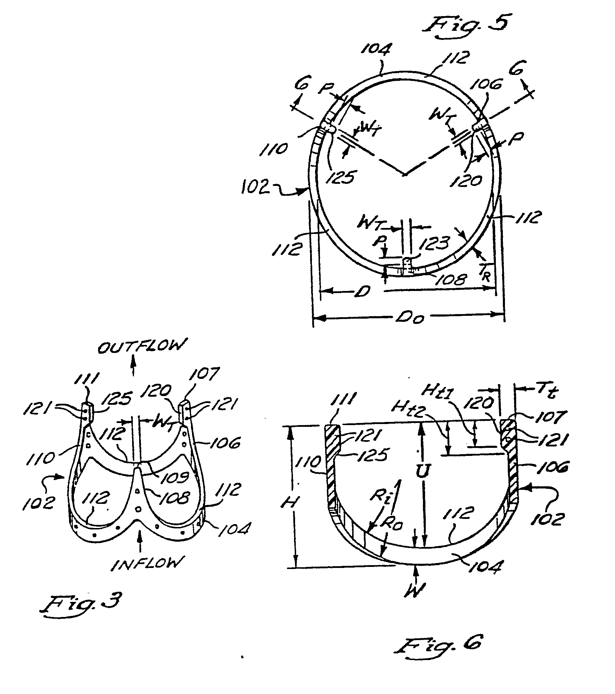

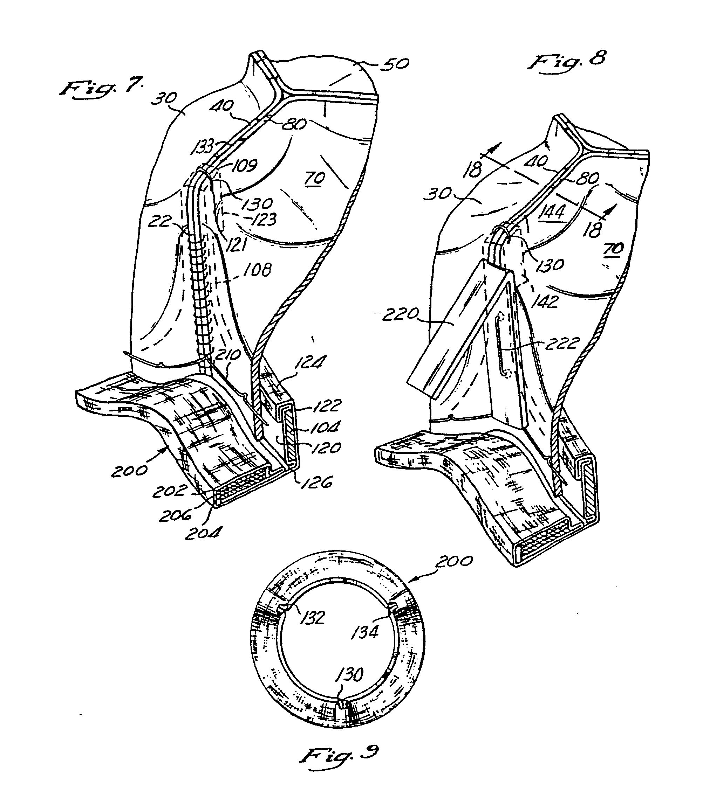

The stent assembly refers generally to the entire stent assembly which includes a biologically compatible metal or plastic stent 102. The stent 102 defines the configuration of the stent assembly. The stent 102 may be considered as three one-third portions of a stent integrally formed of one piece of material although, of course, the method of formation or the number of pieces is of no consequence provided the completed stent is as described herein. The stent 102 comprises an annular ring 104 which extends around and defines the flow orifice of the valve. Coupled to the ring or integrally formed therewith are a plurality of stent legs extending upwardly a distance H toward the outflow end of the valve from the lowermost portion of the base. There are three substantially identical legs 106, 108 and 110, each separated from its neighboring legs by scallops 112 as best shown in Figure 3. The bottom or inflow edge of the stent 102 is also scalloped to conform generally to the arc of the scallops between the legs. These bottom scallops generally follow the configuration of the scallops 112 of the outflow edge so as to generally form parallel edges defining ring 104. The scallops of the lower or inflow edge of the base and the scallops of the outflow edge between the legs vertically define three generally elliptical shaped one-third portions of the base between the centerlines of the respective upright legs which together circumferentially form a right cylinder of constant diameter having an inside diameter D with the legs extending parallel to the axis of the cylinder as shown in Figure 5.

The stent assembly, in the preferred embodiment, also includes a fabric covering which totally or at least substantially encloses the stent 102. It is not essential to the functioning of the present valve that the stent be cloth-covered, but it has been long recognized that there are structural and biological advantages to the use of fully cloth-covered stents for supporting tissue valves. This concept predates the present invention and constitutes no part thereof but is simply adopted as part of the best mode in carrying out the present invention. The fabric covering described in detail by Ionescu et al in U.S. Patent 4,084,268 has been generally adopted, and the same techniques are applied in the present invention as are taught in U.S. Patent 4,084,268 except for the improvements disclosed herein. Reference is made to U.S. Patent 4,034,268 for specific details of the fabrics, knots, sewing and techniques, and the teachings of said patent are incorporated herein by reference. It is sufficient here to describe the stent assembly as including a cloth covering which encloses or substantially encloses and conforms to the stent.

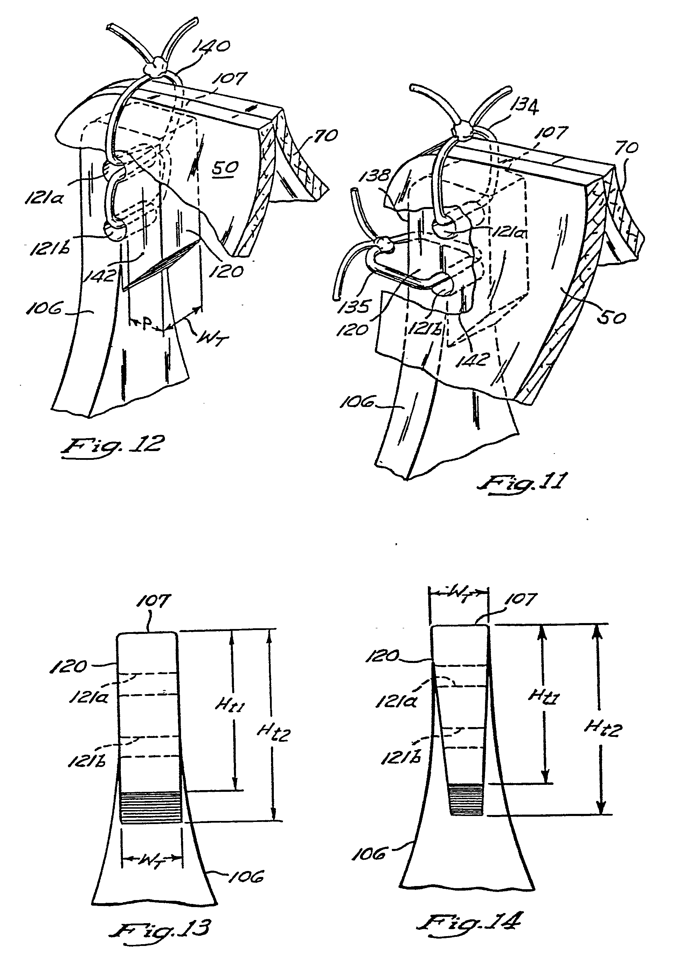

Figure 7 is a partial cross-section depicting a fabric 120 enclosing the outside of the stent a fabric 122 which encloses the inside of the stent, with a suitable seam area 124 joining the fabrics along the top or outflow edge of the stent. A fabric 126 is joined along the lower edge of the stent and extends outwardly forming part of and attaching a sewing or suture ring generally indicated at 200 which may be of any of the forms used in the prior art. Generally, such suture rings comprise a plurality of layers of fabric and padding, 202, 204 and 206, enclosed in layer 126, soft enough to permit the suturing needle to be readily inserted through it and yet rigid and strong enough to provide firm mounting of the prosthesis in the heart valve area. The suture ring 200 differs from the prior art suture rings only in that it curves and conforms to the scalloped contour of the valve ring or base defined by the portion 104 of the stent 102.

The tissue leaflets, after being sewn to form a cylinder as shown in Figure 2, may be attached to the stent assembly in any conventional manner, as, for example, by running stitches shown in 210 in Figure 7.

Figure 7 depicts the valve in a partially completed configuration with the tissue leaflets 30 and 70 joined by seam 22, the upper edges 80 and 40 substantially touching or just touching, without a large or significant surface-to-surface contact of the two leaflets. Coaptation stitch 130 is disposed directly over the tip 109 of the stent leg 108 and passes through a hole 121 in the tab 123 to form the radial coaptation line 133.

Figure 8 shows another stage in the construction of the valve shown in Figure 7 by the addition of the pledget and cover 220. This cover is sewn by stitches 222 to the stent leg through the tissue as described by Ionescu et al in U.S. Patent 4,084,268, or it can be connected in any other convenient manner. The fabric covering, the pledget, and the sewing, all as disclosed with great particularity by Ionescu et al, supra, are utilized in carrying out the invention in its preferred embodiment, but they are not part of the invention per se.

6

**0 125 393**

Coaptation of the tissue leaflets is caused by the action of coaptation stitches 134, 130 and 132 shown in Figure 1. The coaptation of the edges 40 and 60, the edges 40 and 80, as best shown in Figure 18, and the edges 60 and 80 is defined generally by the placement of the coaptation stitches 134, 130 and 132 directly above the respective stent legs 106, 108 and 110, the placement of the coaptation stitch 130 being depicted as exemplary in Figures 7 and 8. In prior art valves such as the one disclosed in U.S. Patent 4,084,268, the coaptation stitch had been placed inside the circumference of the circle defined by the tips of the stent legs. Placement of the coaptation stitch directly above the tips of the stent legs tend to allow the orifice diameter of the fully open valve to equal the inside diameter of the covered stent. Figure 9 illustrates a tissue valve in the fully open position.

Another preferred feature of the heart valve 10 is the relative height H of the stent 102, the depth U of the scallops between the legs of the stent 102, and the inside diameter D of the stent.

In the prior art it was considered necessary, or at least very important, that in smaller valve sizes, e.g. 23 mm or less, the legs be splayed outwardly from the base. Thus, referring to Figure 15, in the prior art stent 102', the input diameter $D_{in}$ was smaller than the outflow diameter $D_{out}$, $D_{out}$ being the diameter of the circle in which the legs at the outflow end of the valve lie. The coaptation stitches of the prior art were formed inside the legs, and the diameter of the circle on which these coaptation stitches were made to lie was made, or attempted to be made, approximately equal to $D_{in}$. Thus, the stent, viewed circumferentially, e.g. from the end, was not a right cylinder, but was generally frustoconical because of the splaying. Sometimes, of course, the splaying of the legs was accomplished by bending the legs out from another wide cylindrical base, but the result was substantially the same as a frustoconical imaginary figure derived from the diameter of the inflow and the outflow ends of the valve.

In contrast to the prior art, the stent 102 of the valve 10 is, viewed circumferentially, a right cylinder, with the axis of the cylinder lying in the center of the flow path and the legs extending from the inflow end toward the outflow end (the top as viewed in the figures) of the valve parallel to the axis of the right cylinder. Thus, $D_{in}$ becomes equal to $D_{out}$.

Also of significance is the ratio U/D, D being equal, of course, to the inner diameter of the stent. As compared with what is regarded as the closest and the most pertinent prior art, the Ionescu type valve described by Ionescu et al in U.S. Patent 4,084,268, various features of which are also described in U.S. Patent No. 4,172,295 to Batten, the scallop depth U measured from the scallop bottom on the outflow edge of the stent ring 104 to the upper or outflow end of the stent legs (see Figure 6), is preferably very much less, for a given valve diameter, than the corresponding distance U' in the prior art stent depicted in Figure 15. In particular, the ratio of U/D in the present valve, more preferably is between about .50 and about .65, and optimally from about .55 to 62.

It is important, of course, to obtain and maintain as low a profile valve as can be made to operate; but that alone is not the only significance of the aforesaid ratio of U/D. This result is obtained by reason of the combination of elements, configurations, relationships, and dimensional ratios, which, acting together, make it possible to provide a heart valve prosthesis, in which the valving element is a generally cylindrical tissue element, which has a profile of less than two-thirds the profile of prior art valves, which closes more rapidly than prior art valves of related construction, and in which the stresses in the cusps of related prior art valves have been wholly or substantially avoided. This result comes about by reason of the interaction and cooperative action and function of the U/D ratio of the stent, the positioning of the coaptation stitch above the end of the stent leg, and the unique configuration of the cusp leaflets of the valving element.

As will be seen in Figure 9, when the valve is in the fully open position, the flow path is substantially a right cylinder through the valve, with the coaptation stitches being placed directly above the legs. This has two functions. The first is of significance but, comparatively, of lesser significance than the other. The first result of this placement of the coaptation stitches is that a larger flow orifice is obtained without the necessity for splaying the legs of the valve. More importantly, the stresses of the prior art tissue valves at four o'clock and at eight o'clock, i.e. at the lower right and the lower left-hand portions of the cusp, when viewing the cusp straight on laterally, have been avoided without fluttering, rolling and floating of the edges of the tissue at the outflow end of the valve. This is an extremely desirable result which follows from the combination of configurations described. This result is accomplished by reason of the configuration of the leaflets in which the valve element comprises three tissue leaflets joined to form a generally cylindrical valve element having three points which extend centrally of each of the cusps respectively toward the outflow end of the valve and centrally between the ends of the legs, the valve element forming in the open position a cylinder having three points on the outflow end thereof and, in the closed position, forming three cusps which are inwardly between the legs with the outflow end of the leaflets touching with the three points adjacent each other in the center of the outflow end of the valve. It will be apparent from a consideration of the structure of the leaflets that, while in the preferred embodiment they are formed of three separate pieces, they may very well be formed of a single integral piece of tissue with appropriate cutting and stitching such that the end result, the valving element, has a proper configuration. Thus, while it is convenient to start with three pieces of tissue, the invention may be practiced with only one piece in which the three leaflets are integrally joined.

The shortening of the implant depth, to less than about two-thirds of the prior art stent heights of corresponding valves, and the adoption of the coaptation stitch directly above the ends of the legs, permit the use of the above described valving element while providing a substantial area of face-to-face

7

overlapping contact along the radial contact lines of the cusps and obviating the tendency of the outflow ends of the leaflets to roll, flutter, and otherwise to delay in closing or to twist and deform by minimizing the coaptation, at the center of the valve, of the plateaus 46, 66 and 86 of the preferred leaflets 30, 50 and 70.

A preferred stent design for tissue heart valves is depicted in detail in Figures 3, 5 and 6. Figure 3 shows the improved stent in perspective. Figure 5 shows a top view of the stent, and Figure 6 is a sectional view of the stent taken along section line 6—6 in Figure 5. It is advantageous to use this preferred stent design in conjunction with the valving element 20 described above. Such a combination is a preferred method of valve construction in the present invention. However, the preferred stent design may be employed with other valving elements, and, on the other hand, the valving element 20 may be used with other stents.

The width $W_t$ of the stent legs 106, 108 and 110 at their tips 107, 109 and 111, as viewed in Figure 5, is substantially less than the width of the tips of prior art stent legs. As shown in Figure 4, which is a detailed sectional view of the tip 107 of stent leg 106, the width $W_t$ is equal to the diameter of the half circle defining the tip if the tip is rounded. In other embodiments where the tip is not rounded, $W_t$ is measured between the points near the tip where the edges of the stent leg first start to curve in toward the center line of the stent leg.

In the preferred embodiment of the stent design, the stent legs are rounded at their tips. The width of the stent legs at the tips thereof has been reduced from approximately 2.032 millimeters in prior stents to a substantially narrower range of widths from about 0.76 mm to about 1.14 mm. This reduced width of the stent legs at their tips has the effect of increasing the radius of curvature of the tissue inside the stent leg and tip. This curvature is caused by the action of the coaptation stitches 134, 130 and 132 in Figure 1 and by closure of the valve which causes collapse of the cusps 30, 50 and 70 toward the center of the valve under restriction of the coaptation stitches. That is, the tissue leaflets 30, 50 and 70 curve less sharply together at the tips 107, 109 and 111 of the stent legs when the tips of the legs are narrower. This increased radius of curvature translates into reduced stress in the tissue of the cusps and a longer service life for the valve.

However, this reduced width of the tips of the stent legs 106, 108 and 110 in Figure 3 leaves less material in the stent legs to carry the structural loading of the closed leaflets caused by the impulse pressure in the flow of blood caused by intermittent pumping action of the heart. To compensate for the reduced width in the leg tips, tabs 120, 123 and 125 (see Figures 3 and 5) are preferably added at the outflow end or tip of each of the stent legs to add structural strength to the legs. These tabs preferably project inwardly toward the center of the stent and are integrally formed with the stent legs and preferably conform to the width $W_T$ at the tips of the stent legs as seen in Figure 5. The tabs extend both vertically down the stent leg for a distance $H_{t1}$, Figure 6, and toward the center of the aperture defined by the stent ring for a distance $T_t$.

The exact dimensions of the tabs, $W_t$, $H_{t1}$, $H_{t2}$ and $T_t$ in Figures 5 and 6, are, within the parameters of this aspect of the invention, matters of design choice. The strength of material used in constructing the stent will affect the choice. Typical dimensions in the preferred embodiment of the stent design are given in Table I below.

The tabs 120, 123 and 125 have apertures 121 formed therein through which the coaptation stitches pass. It is preferred that the coaptation stitches be passed through the holes 121 in the tabs since this registers the position of the coaptation stitch at a positive, repeatable location. The diameter of the holes 121 in the preferred embodiment is 0.813 mm, and the dimension $T_t$ in Figure 6 is chosen to give sufficient strength. The use of holes 121 in the tabs located as described above insures good repeatability of manufacture because different assembly workers cannot change the orifice diameter or induce stresses in the tissue by inadvertent mislocation of the coaptation stitch too far toward or away from the center of the aperture.

The tabs 120, 123 and 125 are included in the preferred embodiment because they have been found to be highly desirable and necessary, in many instances, for sufficient strength; however, the tabs are not always necessary. With refined manufacturing techniques and adaptation of stronger materials, the tabs are expected to be eliminated or reduced in size. The fundamental concept disclosed herein is extension of the service life of the valve by reducing stress levels in the tissue by, among other things, utilizing narrower, preferably rounded, stent leg tips 107, 109 and 111 as shown in Figures 3 and 4. Ideally, a substantially zero tip width would be desirable, but structurally this is impossible at present. The width of the stent leg at its tip is made more narrow than heretofore known; however, the stated width of the tips of the stent legs in the preferred embodiment should not be understood as limiting the invention in any way.

The stent tip should be sufficiently narrow so that the quotient of the radius of curvature of tissue together inside the stent leg tip divided by the thickness of the tissue is greater than or equal to five. The radius of curvature $R_c$ of the tissue inside a typical stent leg tip is illustrated in Figure 4. This curvature is caused by the coaptation of the leaflets under pressure and tends to concentrate shear stresses in the tissue at points 135 and 137 where curvature is greatest. This ratio should be a minimum of approximately five. That is, if the radius of curvature of the tissue around the tip of the stent leg is less than approximately five times the thickness T of the tissue, then the tips of the stent legs are two wide. This ratio has been shown to increase the service life of the valve.

It is critical that the stent dimensions be selected such that touching between the tissue and the stent is substantially eliminated or minimized, and the radius of curvature of the tissue around the stent is not smaller than a predetermined value. That is, touching between the tissue and the stent is substantially eliminated or minimized, and the radius of curvature of the mobile tissue around the stent measured at the point of greatest curvature divided by the thickness of the tissue should be greater than or equal to five. This curvature at two of the various places on the stent where it occurs is illustrated in Figures 4 and 10. Figure 4 is a sectional view taken along section line 4—4 in Figure 2 looking down on the top of the stent leg. The center of the stent is toward the top of Figure 4. The radius lines $R_c$ illustrate the radius of curvature of the tissue 21 together inside the stent leg tip 107 and the fabric covering 113 surrounding the tip. The coaptation stitch 134 is seen to pass through the hole 121 in the stent leg tip and is approximately centered above the tip of the leg. The points of maximum curvature 135 and 137 are seen to be in the tissue at a point just inside of the centermost extremity of the cloth covering 113. The thickness of the tissue is designated as T. The ratio of $R_c/T$ should be greater than or equal to five for extended durability.

Figure 10 is another sectional view of a place of curvature of the tissue around the stent taken along section line 10—10 in Figure 2. Again, $R_c$ indicates the radius of curvature of the tissue 21 over the top of the cloth covering 124 surrounding the base ring 104 of the stent. T indicates the thickness of the tissue and, for extended durability, the ratio $R_c/T$ should be greater than or equal to five.

The reason for the above stated criteria of tip narrowness is that most curvature of the tissue around the stent leg occurs at the tips 107, 109 and 111 of the stent legs where the coaptation stitches 134, 130 and 132 in Figure 1 pull the cusps together. Thus, stress in the tissue is concentrated where the radius of curvature is smallest as can be visualized in examining Figures 7 and 4. Because the tissue formed around the legs and inside of the coaptation stitches is mobile, a large radius through which the tissue can flex helps to reduce the risk of fatigue failures.

Referring to Figure 11, there is shown another embodiment of a coaptation stitch arrangement typical for all three stent legs. This embodiment through the tissue leaflets 50 and 70, through the top hole 121a in the tab 120, and up and over the tip 107 of the stent leg 106. Coaptation stitch 134 could be a group of stitches. Another coaptation stitch 135, or a group of stitches, passes through the tissue leaflets 50 and 70, through the bottom hole 121b in the tab 120, and out and around the outside edge 138 of the stent leg 106. Thus, the top coaptation stitch 134 lies in a plane parallel to the long axis of the stent leg 106. The top stitch 134 or group of stitches is tied off above the tip 107 of the stent leg. The bottom coaptation stitch 135 lies in a plane generally perpendicular to the plane of the top stitch or stitches 134 and is tied off at the outside edge 138 of the stent leg 106. These stitches are placed so that they do not interfere with the normal operation of the valve in the open position.

Because the thread is smaller in diameter than the holes 121, the thread of coaptation stitches 134 and 135 will pull to the side of the holes 121 closest to the stitch knot when the thread is pulled tight. At times during manufacture, the thread may be passed through the tissue leaflets along an axis not parallel to the axis of the bottom hole 121b. Thus, when the thread is pulled tight the tissue leaflet on one side of the hole is moved farther toward the knot at the outside edge 138 of the stent leg 106 than is the tissue leaflet on the other side. Thus uneven pulling can cause wrinkling of the tissue leaflets.

An improvement of the coaptation stitching of Figure 11 is illustrated in Figure 12 which shows the preferred embodiment of the coaptation stitching arrangement. Figure 12 shows a coaptation stitch or stitches 140 residing generally in a plane parallel to the long axis of the stent leg 106 and passing through both the top and bottom holes 121a and 121b and through the tissue leaflets 50 and 70. The stitch can be either a single figure eight stitch as shown in Figure 12 or it can be two separate stitches, each passing through both tissue leaflets and through one of the holes 121a or 121b. Each stitch 140 is tied together above the tip 107 of the stent leg 106. The figure eight stitch shown in Figure 12 is the preferred embodiment, however, because it is simpler and faster to implement than two separate stitches both in a vertical plane. The figure eight stitch is simpler because only one knot need be tied.

The coaptation stitch illustrated in Figure 12 tends to eliminate the tendency for variations in stitch placement during manufacturing which can cause wrinkling of the tissue leaflets.

Referring again to Figure 12, it has been found experimentally that the width, $W_T$, of the tab and the amount of inward projection or protuberance, P, of the tab from the inside edge of the hole toward the center of the flow aperture of the valve is important in preventing abrasion of the tissue leaflets on the inside edges of the tab. When the tissue leaflets coapt together during the closing action of the valve, if the tabs 120, 123 and 125 protrude too far in toward the center of the flow aperture, abrasion can occur in the area generally marked 142 in Figure 8. To prevent this abrasion, the dimension P shown in Figures 5 and 12 is, in the preferred embodiment, restricted to a maximum of forty thousandths of an inch (0.040 inches or 1.016 millimeters). However, any embodiment will be satisfactory wherein the distance which the tab extends towards the center of the aperture is restricted to a distance which substantially eliminates touching between the tissue leaflets and the bottom surfaces or lower 20% of the tab under maximum backpressure conditions. The bottom of the tab surfaces refers generally to those portions of the tab surfaces below the midway point in the height of the tab designated $H_{T2}$ in Figures 6, 13 and 14.

The area of coaptation of the tissue leaflets, designated generally as 144 in Figure 8, tends to grow larger during higher backpressure conditions. This phenomenon can be visualized in placing the fingertips on each hand together fingerprint to fingerprint with the fingertips on the other hand to form a roof shaped arrangement. The fingerprint area of contact represents the area of coaptation 144 in Figure 8. As the hands are pressed together keeping the fingers stiff, the fingers tend to flex toward each other such that the

9

opposing first and second knuckle areas tend to come closer together. This represents the situation when higher backpressure exists on the tissue leaflets during closing.

As the tissue leaflets come closer together under increased backpressure, the area of contact between the leaflets tends to increase by expanding in the downward direction, i.e., toward the stent ring 104 in Figure 7. If the tabs 120, 123 and 125 extend too far in toward the center, abrasion between the mobile areas of the tissue leaflets just inside the tabs and the bottom portions of the tabs can occur. Restriction of the distance which the tabs protrude into the flow aperture tends to eliminate the aforestated abrasion.

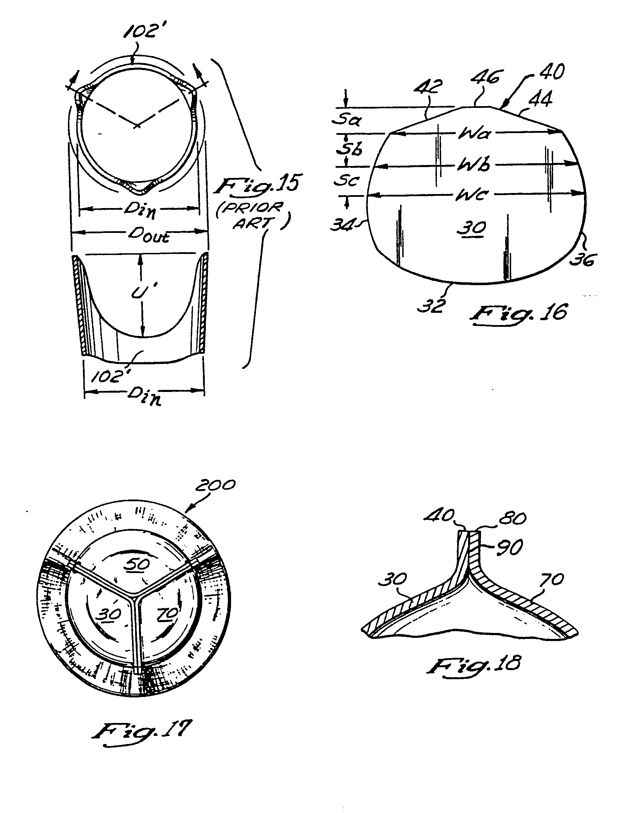

The same reasoning applies to restriction of the relative widths, $W_T$, of the tabs 120, 123 and 125 throughout their height $H_{T2}$ as compared to the relative width of the stent legs 106, 108 and 110 as the stent legs descend from tips 107, 109 and 111. Referring to Figure 13, there is shown a detail view of the preferred embodiment for the tabs and stent leg tips. As seen in Figure 13, the width, $W_T$, of the tab 120 remains constant throughout its height, $H_{T2}$, regardless of the width of the stent leg 106. It is seen in Figure 13 that the width of the stent leg 106 is increasing at points farther away from the tip 107.

Figure 14 shows another embodiment for the tabs wherein the width, $W_T$, of the tab 120 decreases at points farther down from the tip 107 irrespective of the width of said stent leg.

The purpose of maintaining a constant or decreasing width for the tabs 120, 123 and 125 is to minimize the possibility of abrasion of the tissue leaflets on the tabs during closure of the valve and coaptation of the tissue leaflets just inside the tabs. The increasing width of the stent legs 106, 108 and 110 tend to give shape and support to the tissue leaflets to form the cusps of the valve. The increasing width of the stent legs versus the constant or decreasing width of the tabs tends to keep the tissue leaflets away from the lower surfaces of the tabs during coaptation thereby minimizing abrasion. Any shape or form for the tabs which accomplishes the purpose of minimizing or eliminating this abrsion will be satisfactory and is intended to be included within the scope of the claims appended hereto.

As exemplary only and not in any limiting sense, optimum stent dimensions for the stent depicted in Figures 5 and 6 are given in Table I. In Figures 5 and 6, D refers to the inside diameter of the stent ring and $D_o$ refers to the outside diameter thereof. U refers to the depth of scallop 112 and W refers to the width of stent ring 104. $R_i$ refers to the inside radius of the scallop 112 and $R_o$ refers to the outside radius of the scallop forming the bottom of stent ring 104. Finally $H_{T2}$ refers to the total height of the tabs.

It will be apparent that the foregoing description, given in considerable detail as to the method of carrying out the best mode of the invention as contemplated by the inventor, is given to exemplify the concepts and principles of the invention and not to limit it. The stent may be made of titanium, Delrin (TM), polyacetal, polypropylene or Elgiloy with the fabric covering of Dacron or Teflon but the invention is not limited to these materials nor is it limited to any particular covered stent; indeed, the present invention can be carried out without a covered stent. Similarly, the structures and elements of the invention have been described, in their best mode embodiments, as integral, in the case of the stent, and separate, in the case of the leaflets. However, whether formed of one or many pieces, if the structure which results functions in the manner as described herein, it is the same invention. Thus, it is contemplated that the scope of the invention will be as defined in the following claims read in light of the principles of the invention as disclosed herein and not limited by the best mode.

### TABLE 1
#### Stent dimensions (mm) (Page 1 of 2)

| Nominal valve size | Outside diameter $D_o$ (Fig. 5) | Inside diameter D (Fig. 5) | Height H (Fig. 6) | Ring thickness $T_R$ (Fig. 5) | Ring width W (Fig. 6) |
|---|---|---|---|---|---|
| 15 | 13.51 | 11.99 | 10.41 | 0.762 | 2.67 |
| 17 | 15.49 | 13.97 | 11.43 | 0.762 | 2.67 |
| 19 · | 17.53 | 16.00 | 12.45 | 0.762 | 2.92 |
| 21 | 19.56 | 17.78 | 13.46 | 0.889 | 2.92 |
| 23 | 21.336 | 19.56 | 14.48 | 0.889 | 2.92 |
| 25 | 23.37 | 21.34 | 15.75 | 1.016 | 3.18 |
| 27 | 25.40 | 23.36 | 16.76 | 1.016 | 3.18 |
| 29 | 27.30 | 25.02 | 18.03 | 1.143 | 3.43 |
| 31 | 29.51 | 27.23 | 19.25 | 1.143 | 3.43 |
| 33 | 31.24 | 28.96 | 20.62 | 1.143 | 3.68 |

TABLE I
Stent dimensions (mm) (Page 2 of 2)

| Scallop depth U (Fig. 6) | Inside radius $R_i$ (Fig. 6) | Outside radius $R_o$ (Fig. 6) | Leg tip width $W_T$ (Fig. 5) | U/D | Tab thickness $T_T$ (Fig. 6) | Tab Height | |
|---|---|---|---|---|---|---|---|
| | | | | | | HT1 | HT2 |
| | | | | | | (Fig. 6) | |
| 7.74 | 5.13 | 7.42 | 0.761 | 0.646 | 1.65 | 2.79 | 3.56 |
| 8.76 | 6.07 | 8.36 | 0.761 | 0.627 | 1.65 | 2.79 | 3.56 |
| 9.53 | 6.93 | 9.47 | 0.761 | 0.596 | 1.65 | 2.79 | 3.56 |
| 10.54 | 7.72 | 10.26 | 0.889 | 0.593 | 1.78 | 3.20 | 4.06 |
| 11.56 | 8.28 | 10.82 | 0.889 | 0.591 | 1.78 | 3.63 | 4.57 |
| 12.57 | 9.53 | 12.32 | 1.016 | 0.589 | 1.78 | 4.04 | 5.08 |
| 13.59 | 10.29 | 13.08 | 1.016 | 0.582 | 1.78 | 4.44 | 5.59 |
| 14.61 | 11.20 | 14.25 | 1.016 | 0.584 | 1.90 | 4.85 | 6.10 |
| 15.82 | 12.19 | 15.24 | 1.14 | 0.581 | 1.90 | 5.28 | 6.60 |
| 16.94 | 13.21 | 16.51 | 1.14 | 0.585 | 2.03 | 5.69 | 7.11 |

## Claims

1. A prosthetic heart valve comprising:
a) a stent (102) comprising:

1) an annular ring (104) having inflow and outflow edges and defining a flow aperture; and
2) a plurality of legs (106, 108, 110) coupled to said ring (104) and extending from said outflow edge in the direction of flow, with neighboring pairs of said legs (106, 108, 110) being separated by scallops (112) formed in said outflow edge, which scallops (112) are concave towards the direction of flow;

b) a plurality of tissue leaflets (30, 50, 70) attached to said stent (102) and to each other to form a valving element (20); and
c) a plurality of coaptation means (130, 132, 134) in the vicinity of the tips (107, 109, 111) of said stent legs (106, 108, 110) for causing joining of the outflow edges (40, 60, 80) of said tissue leaflets (30, 50, 70), characterized in that said stent (102) is dimensioned such that the quotient of the radius of curvature of mobile tissue (21) divided by the thickness of said tissue (21) is greater than or equal to five at all points of contact between said mobile tissue (21) and said stent (102).

2. The prosthetic heart valve of Claim 1 further characterized in that said valve includes inwardly projecting tab means (120, 123, 125) at the outflow end of each stent leg (106, 108, 110) for adding structural strength to said legs (106, 108, 110), with the distance that said tab means (120, 123, 125) project inwardly toward the center of said flow aperture being restricted to a distance (P) which substantially eliminates abrasion between the tissue leaflets (30, 50, 70) and the bottom surfaces (142) of said tab means (120, 123, 125).

3. The prosthetic heart valve of claim 2 further characterized in that said valve includes means on said tab means (120, 123, 125) coupled to said coaptation means (130, 132, 134) for fixing the position of said coaptation means (130, 132, 134), with said means for fixing the position of said coaptation means comprising a plurality of holes (121) through said tab means (120, 123, 125).

4. The prosthetic heart valve of Claim 3 further characterized in that each of said coaptation means (130, 132, 134) comprises a coaptation stitch (140) lying generally in a plane parallel to the long axis of the stent leg (106) and passing through said plurality of holes (121) and said tissue leaflets (50, 70), and which stitch (140) is tied off above the tip (107) of said stent leg (106), with each of said tabs (120) having two holes (121a, 121b) therein and each of said coaptation stitches (140) defining a figure eight pattern through said two holes (121a, 121b).

5. The prosthetic heart valve of claim 2 further characterised in that the width (Wt) of said tab means (120, 123, 125) remains constant throughout its length while the width of said stent legs (106, 108, 110) increases at points removed from the tips (107, 109, 111) of said stent legs (106, 108, 110).

**Patentansprüche**

1. Prothetische Kerzklappe umfassend:
a) ein Gestell (102) mit:

1) einem kreisförmigen Ring (104) mit Zufluß- und Abflußkanten und eine Strömungsöffnung definierend; und
2) einer Vielzahl von Beinen (106, 108, 110), die mit dem genannten Ring (104) verbunden sind und sich von der genannten Abflußkante in Strömungsrichtung erstrecken mit angrenzenden Paaren der genannten Beine (106, 108, 110), die durch in der genannten Abflußkante gebildete Muscheln (112) getrennt werden, wobei die Muscheln (112) in Strömungsrichtung konkav sind;

b) eine Vielzahl Gewebeplättchen (30, 50, 70), die an dem genannten Gestell (102) und gegenseitig befestigt sind, um ein Klappenelement (20) zu bilden; und
c) eine Vielzahl Koaptationsmittel (130, 132, 134) in der Nachbarschaft der Spitzen (107, 109, 111) der genannten Gestellbeine (106, 108, 110), die die Vereinigung der Abflußkanten (40, 60, 80) der genannten Gewebeplättchen (30, 50, 70) bewirken, dadurch gekennzeichnet, daß das genannte Gestell (102) solchermaßen bemessen ist, daß der Quotient des Krümmungsradiusses des beweglichen Gewebes (21) geteilt durch die Dicke des genannten Gewebes (21) an allen Berührungspunkten zwischen dem genannten beweglichen Gewebe (21) und dem genannten Gestell (102) größer oder gleich 5 ist.

2. Prothetische Herzklappe nach Anspruch 1, weiter dadurch gekennzeichnet, daß die genannte Klappe nach innen abstehende Laschen (120, 123, 125) an dem Abflußende jedes Gestellbeines (106, 108, 110) einschließt, um die Strukturfestigkeit der genannten Beine (106, 108, 110) zu erhöhen, wobei der Abstand, mit dem sich die genannten Laschen (120, 123, 125) nach innen in Richtung des Mittelpunkts der genannten Strömungsöffnung erstrecken auf einen Abstand (P) beschränkt ist, der im wesentlichen einen Abrieb zwischen den Gewebeplättchen (30, 50, 70) und den Bodenflächen (142) der genannten Laschen (120, 123, 125) ausschließt.

3. Prothetische Herzklappe nach Anspruch 2, weiter dadurch gekennzeichnet, daß die genannte Klappe Mittel auf den genannten Laschen (120, 123, 125) einschließt, die mit den genannten Koaptationsmitteln (130, 132, 134) verbunden sind, um die Lage der genannten Koaptationsmittel (130, 132, 134) festzulegen, wobei die genannten Mittel zur Festlegung der Lage der genannten Koaptationsmittel eine Vielzahl Löcher (121) durch die genannten Laschen (120, 123, 125) umfassen.

4. Prothetische Herzklappe nach Anspruch 3, weiter dadurch gekennzeichnet, daß jedes der genannten Koaptationsmittel (130, 132, 134) eine Koaptationsnaht (140) umfaßt, die im allgemeinen in einer, zur langen Achse des Gestellbeins (106) parallelen Ebene leigt und durch die genannte Vielzahl Löcher (121) und die genannten Gewebeplättchen (50, 70) hindurchführt, wobei die Naht (140) oberhalb der Spitze (107) des genannten Gestellbeins (106) abgeschnürt ist und jede der genannten Laschen (120) darin zwei Löcher (121a, 121b) aufweist und jede der genannten Koaptationsnähte (140) ein Achtermuster durch die genannten zwei Löcher (121a, 121b) definiert.

5. Prothetische Herzklappe nach Anspruch 2, weiter dadurch gekennzeichnet, daß die Weite (Wt) der genannten Laschen (120, 123, 125) über deren Länge konstant bleibt, während die Weite der genannten Gestellbeine (106, 108, 110) an von den Spitzen (107, 109, 111) der genannten Gestellbeine (106, 108, 110) entfernten Punkten zunimmt.

**Revendications**

1. Valve cardiaque prothétique comprenant:
a) un cadre (102) comprenant:

1) une bague annulaire (104) possédant des bords d'écoulement d'entrée et d'écoulement de sortie et définissant une ouverture d'écoulement; et
2) plusieurs pieds (106, 108, 110) reliés à ladite bague (104) et s'étendant à partir dudit bord d'écoulement de sortie dans la direction d'écoulement, des paires voisines desdits pieds (106, 108, 110) étant séparées par des festons (112) formés dans ledit bord d'écoulement de sortie, lesquels festons (112) étant concaves dans la direction d'écoulement;

b) plusieurs lamelles tissulaires (30, 50, 70) attachées audit cadre (102) et les unes aux autres pour former un élément valvulaire (20); et
c) plusieurs moyens de coaptation (130, 132, 134) au voisinage des extrémités (107, 109, 111) desdits pieds (106, 108, 110) du cadre afin de provoquer la jonction des bords d'écoulement de sortie (40, 60, 80) desdites lamelles tissulaires (30, 50, 70), caractérisée en ce que ledit cadre (102) est dimensionné de façon une le quotient du rayon de courbure d'un tissue mobile (21) par l'épaisseur dudit tissu (21) soit supérieur ou égal à cinq en tous points de contact entre ledit tissue mobile (21) et ledit cadre (102).

2. Valve cardiaque prothétique selon la revendication 1, caractérisée en outre en ce que ladite valve comporte des pattes (120, 123, 125) faisant saillie vers l'intérieur à l'extrémité d'écoulement de sortie de chaque pied (106, 108, 110) du cadre afin d'ajouter à la résistance structurelle desdits pieds (106, 108, 110), la distance sur laquelle lesdites pattes (120, 123, 125) font saillie vers l'intérieur en direction du centre de ladite ouverture d'écoulement étant réduite à une distance (P), ce qui élimine sensiblement l'abrasion entre les lamelles tissulaires (30, 50, 70) et les surfaces inférieures (142) desdites pattes (120, 123, 125).

3. Valve cardiaque prothétique selon la revendication 2, caractérisée en outre ce que ladite valve comprend des moyens situés sur lesdites pattes (120, 123, 125), couplés auxdits moyens de coaptation (130, 132, 134) afin de fixer la position desdits moyens de coaptation (130, 132, 134), lesdits moyens pour fixer la position desdits moyens de coaptation comprenant plusieurs trous (121) traversant lesdites pattes (120, 123, 125).

4. Valve cardiaque prothétique selon la revendication 3, caractérisée en outre en ce que chacun desdits moyens de coaptation (130, 132, 134) comprend un point de coaptation (140) s'étendant globalement dans un plan parallèle au grand axe du pied (106) de cadre et passant dans lesdits plusieurs trous (121) et à travers lesdites lamelles tissulaires (50, 70), et lequel point (140) est noué au-dessus de l'extrémité (107) dudit pied (106) du cadre, chacune desdites pattes (120) présentant deux trous (121a, 121b) et chacun desdits points de coaptation (140) définissant la forme d'un chiffre huit dans lesdits deux trous (121a, 121b).

5. Valve cardiaque prothétique selon la revendication 2, caractérisée en outre en ce que la largeur (Wt) desdites pattes (120, 123, 125) reste constante sur toute leur longueur, tandis que la largeur desdits pieds (106, 108, 110) du cadre augmente en des points éloignés des extrémités (107, 109, 111) desdits pieds (106, 108, 110) du cadre.

*Fig. 1*

*Fig. 2*

CENTER
OF STENT

*Fig. 4*

*Fig. 10*

Fig. 5

OUTFLOW

INFLOW

Fig. 3

Fig. 6

Fig. 7

Fig. 8

Fig. 9

0 125 393

*Fig. 12*

*Fig. 11*

*Fig. 13*

*Fig. 14*

*Fig.15*
(PRIOR ART)

*Fig.16*

*Fig.17*

*Fig.18*